# EUROPÄISCHE PATENTSCHRIFT

(11) **EP 1 984 739 B1**
(45) Veröffentlichungstag und Bekanntmachung des Hinweises auf die Patenterteilung: **11.08.2010**
(21) Anmeldenummer: 07703798.4
(22) Anmeldetag: 11.01.2007
(51) Int. Cl.: G01N 33/543, G01N 33/58

(54) **VERFAHREN UND DIAGNOSTISCHES KIT ZUR SIMULTANEN IDENTIFIZIERUNG UND QUANTIFIZIERUNG VON EINZELNEN ODER IN GEMISCHEN VORLIEGENDEN BIOMOLEKÜLEN UND DEREN MODIFIKATIONEN**
METHOD AND DIAGNOSTIC KIT FOR SIMULTANEOUS IDENTIFICATION AND QUANTIFICATION OF INDIVIDUAL BIOMOLECULES OR BIOMOLECULES WHICH ARE PRESENT IN MIXTURES AND THEIR MODIFICATIONS
PROCEDE ET KIT DE DIAGNOSTIC POUR L'IDENTIFICATION ET LA QUANTIFICATION SIMULTANEE DE BIOMOLECULES ISOLEES OU EN MELANGE ET DE LEURS MODIFICATIONS

(30) Priorität: 12.01.2006 DE 102006001793
(43) Veröffentlichungstag der Anmeldung: 29.10.2008
(73) Patentinhaber: Leibniz - Institut für Analytische Wissenschaften - ISAS - E.V., 44139 Dortmund (DE); Forschungsgesellschaft für Arbeitsphysiologie und Arbeitsschutz e.V., 44139 Dortmund (DE)
(72) Erfinder: JAKUBOWSKI, Norbert, 44319 Dortmund (DE); ROOS, Peter H., 45549 Sprockhövel (DE)
(74) Vertreter: Zimmermann & Partner
(86) Internationale Anmeldenummer: PCT/EP2007/050254
(87) Internationale Veröffentlichungsnummer: WO 2007/082833

(56) Entgegenhaltungen:
- US-A1- 2002 055 186
- US-A1- 2005 069 911
- US-A1- 2006 223 130
- QUINN Z A ET AL: "Simultaneous determination of proteins using an element-tagged immunoassay coupled with ICP-MS detection" JOURNAL OF ANALYTICAL ATOMIC SPECTROMETRY, NEW YORK,NY, US, Bd. 17, Nr. 8, 2. Juli 2002 (2002-07-02), Seiten 892-896, XP002973532

## Beschreibung

### Technisches Gebiet

Die Erfindung betrifft ein Verfahren zur simultanen Bestimmung der Identität und/oder Quantifizierung von Biomolekülen und mindestens einer ihrer Modifikationen. Die Erfindung betrifft ferner ein diagnostisches Kit zur Identifizierung und Quantifizierung von Biomolekülen mit einem solchen Verfahren. Die Erfindung betrifft insbesondere die gleichzeitige Identifizierung und Quantifizierung von Proteinen und ihrer posttranslationalen Modifikationen.

### Stand der Technik

Das genetische Material einer Zelle oder eines Organismus wird als Genom bezeichnet. Es liegt in jedem Entwicklungszustand des Organismus unverändert vor. Durch die Möglichkeit der Sequenzierung von Nukleinsäuren und Polymerasekettenreaktion ist es heute möglich, Genome vollständig zu analysieren.

Um komplexe physiologische Vorgänge im Organismus zu verstehen, reicht es jedoch nicht aus, das Genom zu entschlüsseln. Es ist notwendig, die Proteine und andere Biomoleküle in ihrer Gesamtheit zu erfassen. Denn die über die Gene verschlüsselten Proteine, die in einer Zelle oder einem Gewebe exprimiert wurden, sind die eigentlichen biologischen Effektormoleküle. Sie bestimmen das biologische Geschehen, steuern dynamische Prozesse und führen die verschiedensten Funktionen aus.

Die Biosynthese von Proteinen (Translation) ist gewebs- bzw. zellspezifisch. Jedoch sind äußere Bedingungen, wie zum Beispiel Temperatur, Stress, Interaktionen mit anderen Zellen, Medikamente oder Toxine dafür ausschlaggebend, welche Proteine und in welcher Menge diese synthetisiert werden. Das Proteom ist daher ein dynamisches, sich ständig änderndes Gebilde. Nach der Translation können die Proteine durch verschiedene sogenannte posttranslationale Modifikationen weiter verändert werden und so ihre Wirkungsweise ändern.

Die meisten dieser Modifikationen werden durch den Organismus bzw. durch die Zellen selbst ausgelöst. Diese Prozesse können konstitutiv ablaufen oder aber durch Umwelteinflüsse oder andere Parameter beeinflusst werden. Diese Modifikationen sind oft kovalente Bindungen von funktionellen Gruppen an eine oder mehrere Aminosäuren des Proteins.

Zu den posttranslationalen Modifikationen zählen u. a. folgende Vorgänge, die zu neuen Proteinspezies führen: die Abspaltung des N-terminalen Formylrestes von Formylmethionin, die Abspaltung des Methionylrestes am N-Terminus neusynthetisierter Proteine, Phosphorylierungen, Glykosylierungen, das Knüpfen von Disulfidbrücken, die Veränderung der Faltung durch Chaperone, die gezielte Abspaltung von Signal- oder von anderen Teilsequenzen, die Verknüpfung mit Coenzymen und prosthetischen Gruppen, die Bindung von Ionen und niedermolekularen Substanzen, die Bildung von Proteinkomplexen, die mRNA-unabhängige Synthese von Polypeptiden mit Hilfe von Enzymen, die Proteininaktivierung und -fragmentierung durch Proteolyse, die Ubiquitinierung, die Acetylierung, die Methylierung, die Modifikation mit Fettsäureresten.

Es sind nur etwa 30 posttranslationale Modifikationen bekannt. Ihre wirkliche Anzahl ist jedoch wesentlich größer. Die Kenntnis dieser Modifikationen ist neben der Identifizierung der zugehörigen Proteine der Schlüssel zum Verständnis der intermolekularen Wechselwirkungen im Organismus.

Es sind zahlreiche Methoden zur Identifizierung und Quantifizierung von Proteinen oder Proteinmischungen bekannt. Diese beschränken sich häufig auf die Identifizierung des Proteins, ohne seine posttranslationalen Modifikationen zu erkennen. Diese sind aber wichtig, um die Reaktionsweisen des Proteins, seinen Aktivitätszustand oder seine Komplexbildung mit anderen Molekülen zu erkennen.

Eine übliche Methode der Proteomanalyse, die es ermöglicht, Proteine aus dem Proteom voneinander zu trennen, ist die 2D-Gelelektrophorese (2D-SDS-PAGE), bekannt aus der Veröffentlichung P. H. O'Farrell, J. Biol. Chem., 1975, 250, 4007-4021. An die Elution der Proteine aus dem Gel schließt sich die Identifizierung und Charakterisierung der getrennten Spezies an. Die Identifizierung erfolgt zum Beispiel mittels massenspektrometrischer Methoden.

Zahlreiche Proteindatenbanken gestatten dann im Zusammenhang mit Suchkriterien wie der Herkunft des Proteins, seinem Molekulargewicht, der eingesetzten Protease zur Spaltung des Proteins in Peptide die eindeutige Identifizierung des Proteins.

Vor einer 2D-Gelelektrophorese ist häufig die Anreicherung des Proteins zu seiner Messung erforderlich. Außerdem ist dieses Verfahren zeit- und arbeitsaufwendig. Die nicht sehr gute Reproduzierbarkeit des Gels macht es schwierig, zwischen einer Änderung des Elektrophoresesystems und induzierter biologischer Modifikation zu unterscheiden.

Aus M. Uenlue, M. E. Morgan and J. S. Minden, Electrophoresis, 1997, 18, 2071-2077 ist die 2D-Differenz-Gelelektrophorese (DIGE) bekannt. Dabei wird mehr als eine Probe in einem Polyacrylamidgel separiert. Bei dieser Technik müssen zwei Proteinextrakte vormarkiert werden, wobei die Fluoreszenzfarbstoffe cy3 und cy5 eingesetzt werden.

Es ist weiterhin die bioanorganische Analyse von heteroatom- und bevorzugt metallmarkierten Proteinen bekannt. Die Heteroatome in den Proteinen, wie z. B. P, S, Se, Cd und andere Metalle können mit Atomspektroskopie und insbesondere mit LC(liquid chromatography)-gekoppelten ICP(inductively coupled plasma)-MS(mass spectrometry)-Bestimmungen nachgewiesen werden. Weiterhin ist die Detektion mit Laserablations-ICP-MS nach der Trennung des Proteingemisches durch Gelelektrophorese bekannt. Dieses Verfahren diente der Detektion von phosphorylierten Proteinen.

Es ist ferner in der quantitativen Proteomanalyse der Einsatz von isotopen- oder metallcodierten Affinitätstags (ICAT und MeCAT) bekannt. Ein Affinitätstag erlaubt die spezifische, reversible Kopplung von Proteinen an ein Trägermaterial. In der anorganischen Massenspektrometrie, insbesondere ICP-MS, sind Markierungen für Verbindungen angewendet worden, die selbst kein detektierbares Heteroelement enthalten. Dabei wurden Alkohole mit Si markiert und Carbonsäuren in pharmazeutischen Proben unter Nutzung von phosphorhaltigen Verbindungen.

Das Markieren von Proteinen durch Metalle und besonders durch Lanthanide und die Detektion durch ICP-MS ist ebenfalls bereits offenbart. Bekannt ist das Markieren von Antikörpern mit Goldnanoclustern in Verbindung mit der Detektion durch Laserablations-ICP-MS auf Blot-Membranen. Fluoreszierende Proben, die Eu, Tb, Dy und Sm enthalten, wurden genutzt, um Antikörper zu markieren und durch ICP-MS in flüssigen Proben zu detektieren.

Die DE 102 27 599 A1 offenbart ein verbessertes CAT-basiertes Verfahren und das entsprechende CAT-Reagenz, das eine Anwendung in der Hochdurchsatzumgebung erlaubt. Das Verfahren dient zur Identifizierung und Quantifizierung von einem oder mehreren Proteinen in einem Proteingemisch mittels eines CAT-Reagenzes vom Typ A-Y-PRG, worin A eine funktionelle Gruppe zur reversiblen, kovalenten oder nichtkovalenten Bindung an ein Trägermaterial darstellt. Y ist eine Gruppe, die mindestens eine Chelatfunktion für isotopenarme Metalle und ein daran gebundenes Metallion umfasst. PRG ist eine reaktive Gruppe zur selektiven Bindung an Peptide oder andere Biomoleküle. Zunächst werden die Proteine der Probe in Peptide gespalten. Dann werden die Peptide an das CAT-Reagenz gebunden. Die markierten Peptide binden an das Trägermaterial und werden so vom Rest der Mischung getrennt. Die Peptide werden eluiert und dann mit Hilfe von Massenspektrometrie nachgewiesen und charakterisiert. In dem Molekül A-Y-PRG wird der Y-Teil u. a. von DOTA (1,4,7,10-Tetraazacyclododekan-1,4,7,10-Tetraessigsäure) und Derivaten davon und DTPA (Diethylentriaminpentaessigsäuredianhydrid) gebildet.

Die US 2005/0059100 A1 offenbart Verbindungen und Methoden, um Biomoleküle zu detektieren, zu analysieren und zu identifizieren. Insbesondere beschreibt die Veröffentlichung elementkodierte Affinitäts-Tags, die ein Metallchelat und ein Metallion beinhalten. Dabei werden zwei Proben mit jeweils unterschiedlichen Tags kontaktiert, wobei die Mengen an Addukt in einer massenspektrometrischen Vergleichsmessung bestimmt werden. Verschiedene Chelatliganden werden vorgeschlagen, darunter auch DOTA- und DTPA-Derivate. Die Bindung der Addukte an Affinitätsmedien wird beschrieben.

Die US 2004/0072250 A1 offenbart Methoden zur Detektion und Messung von elementmarkierten biologisch aktiven Materialien. Zur Detektion der markierten Spezies dienen Atommassen- oder optische Spektrometer mit Atom- oder Atomionenquellen (u. a. Laserablations-ICP-MS). Elementmarkierte Materialien, u. a. Antikörper, Proteinkomplexe und Hormone, die mit dem Elementtag verbunden sind, können in Binding-Assays genutzt werden und durch elementspektroskopische Detektion gemessen werden.

Es werden auch Methoden zur Bestimmung von Metallen in Proben durch Nutzung spezifischer Antikörper zur Isolierung der Zielmetalle beschrieben. Das markierte biologisch aktive Material bildet mit mindestens einem Analyten oder Analytkomplex (ein an andere Moleküle oder biologisch aktive Materialien gebundener Analyt) ein Addukt. Das gebundene markierte Material wird vom ungebundenen Material getrennt und die gebundene markierte Spezies wird mit einem Atommassen- oder optischen Spektrometer detektiert und gemessen. Der Weg zum Erhalt des Adduktes kann die Schritte Markierung, Komplexbildung mit dem Analyten, Abtrennung des gebundenen Materials etc. in verschiedener Reihenfolge enthalten.

Ausgestaltungen der Methoden sehen die Komplexierung von Antikörpern mit Elementspezies (ein an ein anderes Atom oder an eine Atomgruppe gebundenes Element) vor, die dann gemessen werden. Weiterhin wird die Detektion und Quantifizierung eines Analyten durch direktes Markieren des Analyten beschrieben. Eine andere Variante zeigt die Bindung des biologisch aktiven Materials an den Analyten der Probe. Das zu messende Element befindet sich als Tag auf einem sekundären biologisch aktiven Material, das mit dem abgetrennten primären an den Analyten gebundenen biologischen Material kombiniert wird. Der gebildete Komplex wird abgetrennt und vermessen. Letzteres Vorgehen wird erweitert durch die Einführung eines dritten markierten biologisch aktiven Materials, das an einen Komplex aus erstem biologisch aktivem Material, Analyt und zweitem biologisch aktiven Material bindet.

Außerdem werden Kits vorgestellt, die alle nötigen Reagenzien und Anweisungen enthalten, um die oben aufgeführten Methoden durchzuführen. Es wird auch die Möglichkeit eingeschlossen, unterschiedlich markierte biologisch aktive Materialien mit unterschiedlichen Analyten zu komplexieren und so mehrere Analyten simultan zu vermessen. Allen bekannten Methoden gemeinsam ist es, dass jeweils nur eine Komponente des gebildeten Adduktes, das zur Vermessung gelangt, markiert ist. Das Ergebnis der Messung ist jeweils die Identifizierung des Analyten an sich, seine eventuellen Modifikationen werden nicht erkannt.

Quinn et al. offenbaren im J. Anal. At. Spectr., 2002, 17, 892 - 896 eine Methode zur simultanen Quantifizierung von mehreren Proteinen in komplexen biologischen Proben. Die Verwendung von differenzierbaren mit Element-Tags markierten Antikörpern gestattet die Diskriminanzdetektion mit einem ICP-Massenspektrometer.

Die US 2002/0055186 offenbart eine Methode und Vorrichtungen zur Bestimmung von Proteinen. Die Proteine werden in Peptidfragmente zerlegt und ggf. markiert. Die Peptidfragmente werden dann mit auf einer Trägeroberfläche immobilisierten Molekülen, z. B. Antikörpern, kontaktiert. Diese Moleküle erkennen die Peptidfragmente und binden sie spezifisch. Es werden Methoden zur Herstellung derartiger Arrays beschrieben. Erwähnt ist weiterhin die Bestimmung von posttranslationalen Modifikationen der Peptidfragmente durch z. B. massenspektrometrische Analyse.

Die US 2005/0069911 A1 beschreibt Methoden zur verlässlichen Bestimmung von Proteinen, die Träger verschiedener posttranslationaler Modifikationen sein können. Es wird eine vorangehende Proteolyse der Proteine beschrieben.Ein oder mehrere proteinerkennende und proteinbindende Arrays werden mit diesen Proteinen kontaktiert. Diese Spezies sind selektiv für URSs (Unique Recognition Sequences) oder PETs (Proteome Epitope Tags), die wiederum typisch für bestimmte Proteine sind. Eine Detektion der posttranslationalen Modifikationen wird ebenfalls beschrieben. Dazu wird die Aminosäurensequenz des Proteins bestimmt, um mögliche Positionen für posttranslationale Modifikationen zu ermitteln. Es wird die Bestimmung des Aktivierungszustands von Signalwegen als Antwort auf eine Stimulation dargelegt.

Die Nutzung der Laserablations-ICP-MS zur Detektion von biologisch aktiven Molekülen ist bekannt. Zu diesem Zweck ist eine optimierte Laserablationszelle für Ablationsmessungen mit der Verwendung von Membranen großer Oberfläche bekannt. Diese Zelle wurde angewendet für den quantitativen Phosphor-Nachweis in phosphorylierten Proteinen.

Alle bekannten Verfahren sind in der Regel aufwendige Hestimmungsmethoden, denen in der Regel komplexe Trennverfahren oder Aufspaltungen der Proteine in Peptide vorausgehen müssen. Oft müssen die Analyten vor der Vermessung angereichert werden.

Häufig ist die Kombination verschiedener Trenn- und Nachweismethoden nötig. Die Simultanbestimmung einer großen Anzahl von Analyten ist nur eingeschränkt möglich.

### Offenbarung der Erfindung

Es ist Aufgabe der vorliegenden Erfindung, ein Verfahren der eingangs genannten Art zu schaffen, das einfacher ist und mit dem die simultane Bestimmung von Biomolekülen, insbesondere Proteinen, und deren Modifikationen im Hochdurchsatzverfahren möglich ist.

Erfindungsgemäß wird die Aufgabe durch ein Verfahren der eingangs genannten Art gelöst mit den Schritten gemäß Anspruch 1.

Es versteht sich, dass auch eine andere Reihenfolge der Schritte denkbar ist.

Unter dem zu identifizierenden und quantifizierenden Biomolekül ist hier jedes Molekül zu verstehen, das biologisch aktiv ist oder sein kann, so zum Beispiel Proteine, Nukleotide, Rezeptoren, Lektine, Oligo- und Polysaccharide, Lipopolysaccharide, Zellmetaboliten, Hormone, pharmakologisch aktive Substanzen, Alkaloide, Steroide, Vitamine, Aminosäuren und Zucker. Die Proteine umfassen u. a. Antigene, Antikörper, Immunoglobuline, Enzyme, Lipoproteine, Glykoproteine, Peptide und Polypeptide. Die Nukleotide umfassen u. a. Oligo- und Polynukleotide, DNA und RNA. Unter den Begriff Biomolekül fallen hier nicht nur natürliche sondern auch synthetisch hergestellte Biomoleküle. Unter synthetisch hergestellten Biomolekülen werden hier Kopien von natürlich vorkommenden Biomolekülen und nicht natürlich vorkommenden Biomolekülen verstanden. Letztere sind zum Beispiel Modellsubstanzen für natürlich vorkommende Biomoleküle, die in der medizinischen Forschung eingesetzt werden.

Die Biomoleküle können einzeln vorliegen oder in Mischungen. Sie können aus menschlichen, tierischen oder pflanzlichen Proben, beispielsweise aus Körperflüssigkeiten, Zelllysaten, Pflanzenextrakten, Organsekreten stammen. Es kann sich auch um Fraktionen aus chromatographischen, gelelektrophoretischen oder anderen Trennungen der verschiedensten Biomolekülmischungen handeln.

Unter Element-Tag wird hier eine Markierungsspezies verstanden, die an ein Biomolekül oder eine modifikationserkennende Spezies gebunden werden kann und die ein analytisch differenzierbares Element enthält oder selbst ein solches Element ist. Im Falle von Halogenierungen (z. B. Iodierungen von Histidin- und Tyrosin-Resten in Proteinen) ist das Elementtag ein Halogen-Atom. Element-Tags können auch Atomgruppen oder Moleküle (zum Beispiel Metallkomplexe) sein, die mindestens ein beliebiges analytisch differenzierbares Element enthalten.

Unter biomolekülerkennender Spezies wird jedes Molekül verstanden, das ein zu untersuchendes Biomolekül erkennt und spezifisch bindet. Im bevorzugten Fall, dass es sich bei dem Biomolekül um ein Protein handelt, ist die molekülerkennende Spezies der zugehörige spezifische Antikörper.

Unter einer Modifikation des Biomoleküls werden hier alle seine funktionellen Gruppen und mit dem Biomolekül verknüpfte Atome, Atomgruppen oder Moleküle verstanden. Modifikationen umfassen zum Beispiel Zuckermoleküle, Metalle, Phosphatgruppen, Ubiquitin, Toxine, Metaboliten verschiedenster Herkunft, Iodierungen. Die Aufzählung ist nicht vollständig. Für den bevorzugten Fall, dass es sich bei dem Biomolekül um Proteine handelt, zählen zu den Modifikationen die posttranslationalen Modifikationen. Unter modifikationserkennendem Molekül wird jedes Molekül verstanden, das selektiv nur eine bestimmte Modifikation des Biomoleküls erkennt und mit dieser eine feste kovalente oder nichtkovalente Bindung eingeht. In einer bevorzugten Ausgestaltung der Erfindung sind die Biomoleküle Proteine und die biomolekülerkennenden Spezies die zugehörigen Antikörper. Für die Erkennung von Modifikationen in Form von Glykolysierungen kommen als modifikationserkennende Moleküle Lektine in Frage, für die Erkennung von Ubiquitinierungen anti-Ubiquitin, für SUMO(small ubiquitin-related modifier protein)-Anlagerungen anti-SUMO. Das erfindungsgemäße Verfahren ist auf viele denkbare Modifikationen des Biomoleküls übertragbar und lässt das breite Verwendungspotential des Verfahrens erkennen.

Ein typisches Vorgehen nach dem erfindungsgemäßen Verfahren sieht die Immobilisierung von biomolekülerkennenden Spezies auf einer geeigneten Oberfläche vor. Die zu analysierenden Biomoleküle werden mit Element-Tags markiert und mit der Oberfläche kontaktiert. Die biomolekülerkennenden Spezies binden spezifisch die zugehörigen Biomoleküle. In einem weiteren Schritt werden modifikationserkennende Moleküle mit anderen spezifischen, analytisch eindeutig differenzierbaren Tags markiert. Es sind auch spezielle Kombinationen ausgewählter, mit differenzierenden Tags versehener modifikationserkennender Moleküle für ein umfassenders Multiplexing denkbar. Die so vorbereiteten modifikationserkennenden Moleküle werden mit den an die biomolekülerkennende Spezies gebundenen und damit an der Oberfläche indirekt immobilisierten Biomolekülen in Kontakt gebracht. Ist das Biomolekül Träger der Modifikation, die dem modifikationserkennenden Molekül entspricht, bindet letzteres an die zugehörige Modifikation. Es liegt dann ein System aus molekülerkennender Spezies, Biomolekül mit Modifikation und modifikationserkennender Spezies vor. Die Bestimmung der Identität und die Quantifizierung der vorhandenen Tags ermöglicht die Identifizierung und Quantifizierung des Biomoleküls und seiner Modifikation. Es können auch mehrere Modifikationen gleichzeitig bestimmt werden. In diesem Fall binden mehrere modifikationserkennende Spezies an dasselbe Biomolekül, das Träger verschiedener bzw. mehrfach auftretender identischer Modifikationen ist.

Im Gegensatz zu bekannten Verfahren werden hier keine aufwendigen Trennungsschritte mittels Chromatgraphie oder Elektrophorese benötigt. Die Erfindung basiert auf der Erkenntnis, dass sowohl Element-Tags der Biomoleküle als auch analytisch eindeutig differenzierbare modifikationserkennende Moleküle simultan in einem einzigen Detektionsschritt bestimmt werden können.

Ein weiterer Vorteil dieses Verfahrens besteht in der Tatsache, dass eine große Vielzahl von Biomolekülen und ihrer Modifikationen gleichzeitig bestimmt werden kann. Das Verfahren gestattet auf einer Oberfläche die Identifizierung und Quantifizierung von mehreren Biomolekülen bzw. Biomolekülgemischen und der zugehörigen Modifikationen nebeneinander. Das Verfahren zur gleichzeitigen Bestimmung von Biomolekülen und ihrer Modifikationen ist im Gegensatz zu bekannten Verfahren im Hochdurchsatz durchführbar. Das sogenannte "Multiplexing" zur Darstellung von Unterschieden verschiedener Biomoleküle und/oder Biomolekülgemische auf einer einzigen Oberfläche ist hier potenziert worden. Es wurde erweitert um die zusätzliche, gleichzeitige Darstellung von Unterschieden zwischen den Arten der Biomoleküle selbst neben der Darstellung von Unterschieden in den Modifikationen innerhalb eines Biomoleküls und der Darstellung von Unterschieden in den Modifikationen mehrerer Biomoleküle im Vergleich zueinander.

Vorzugsweise kann auf der Oberfläche eine Vielzahl von biomolekülerkennenden Spezies immobilisiert werden. Das hat den Vorteil, dass das Verfahren die gleichzeitige Analyse von einer Vielzahl von Proben und Biomolekülen gestattet. Hochdurchsatzanalysen von komplexen Biomolekülgemischen bei gleichzeitiger Erfassung von Biomolekülmodifikationen sind möglich. Bisher war zum Erhalt dieser Ergebnisse, zum Beispiel der Identifizierung des Proteins und seiner posttranslationalen Modifikationen, eine Kombination von Methoden mit verschiedenen zeit- und arbeitsaufwändigen Arbeitsgängen und Trennverfahren nötig.

In einer besonders bevorzugten Ausgestaltung der Erfindung ist die Oberfläche eine Mikroarray-Oberfläche. Sie kann aus Metallclustern, Metallen, organischen oder anorganischen Polymeren, Glas oder Halbleitern bestehen. Es sind auch andere Substrate verwendbar. Auf einem Mikroarray bzw. Chip (z.B. im Objektträgerformat 26 mm x 76 mm) können bis zu mehreren Tausend molekülerkennende Spezies an definierten und automatisch ansteuerbaren Positionen, sogenannten Spots immobilisiert werden. Als Oberfläche kann natürlich auch eine Makroarray-Oberfläche verwendet werden.

Biomolekülerkennende Spezies können oft über ihre funktionelle Gruppen direkt auf der Oberfläche verankert werden, indem sie mit reaktiven Gruppen des Oberflächensubstrats kovalente Bindungen eingehen. Andere molekülerkennende Spezies können auch oder nur mit Hilfe von Linkern an die Mikroarrayoberfläche gebunden werden.

Besonders bevorzugt ist insbesondere in der Proteinanalyse eine Goldoberfläche. Die biomolekülerkennenden Spezies im Fall von Proteinen sind in der Regel Antikörper, die mit Hilfe von Linkern auf der Oberfläche des Mikroarrays gebunden werden. Besonders geeignet sind in diesem Zusammenhang thiolgruppenfunktionalisierte Linker, da sie leicht fest auf der Goldoberfläche verankert werden können.

Diese Mikroarrays mit speziell definierten Bindungsstrukturen für zu analysierende Biomoleküle sind kommerziell erhältlich. Sie können vom Fachmann mit den üblichen Techniken aber auch selbst hergestellt werden und mit individuellen biomolekülerkennenden Spezies versehen werden. Die markierten Biomoleküle werden selektiv an die zuvor oberflächenimmobilisierten biomolekülerkennenden Spezies gebunden. Die biomolekülerkennenden Spezies und ihre genauen Positionen auf dem Mikroarray sind bekannt und können zur Detektion der dort gebundenen Biomoleküle gezielt angesteuert werden. Die Kenntnis der angesteuerten biomolekülerkennenden Spezies verbunden mit dem Nachweis des zur Markierung des gebundenen Biomoleküls verwendeten Element-Tags ist ein eindeutiger Hinweis auf die Identität des Biomoleküls.

Das Verfahren kann auch einen doppelten Beweis für die Identität eines Biomoleküls liefern. Das an seine biomolekülerkennende Spezies gebundene markierte Biomolekül wird dazu erneut mit der biomolekülerkennenden Spezies in Kontakt gebracht, die diesmal mit einem anderen Element-Tag markiert ist als dieses Biomolekül. Das Biomolekül wird sandwichartig zwischen zwei biomolekülerkennenden Spezies eingeklemmt. Die Detektion beider zur Markierung eingesetzten Elemente ist ein eindeutiger Beweis für das Vorhandensein des Biomoleküls, für das die biomolekülerkennende Spezies spezifisch ist. Die doppelte unspezifische Bindung der molekülerkennenden Spezies an ein "falsches" Biomolekül ist sehr unwahrscheinlich und statistisch gesehen auszuschließen. Es liegt ein klarer Beweis für die Identität des Biomoleküls vor.

Durch das neue Verfahren in Form eines Multiplexing im Mikroformat (Mikroarrays) können in einem Arbeitsgang viele Informationen aus einer immobilisierten Probe gewonnen werden.

Damit das modifikationserkennende Moleküle in der Analyse eindeutig identifiziert werden kann, muss es analytisch differenzierbare Eigenschaften aufweisen. Ist das Molekül nicht schon von Natur aus Träger derartiger Eigenschaften, bei denen es sich u. a. um enthaltene Heteroatome, chromophore Gruppen oder fluoreszierende Struktureinheiten handeln kann, muss das modifikationserkennende Molekül vor dem Kontaktieren mit den Biomolekülen mit spezifischen, analytisch differenzierbaren Tags markiert werden. Unter spezifischen, analytisch differenzierbaren Tags werden unter anderem Element-Tags verstanden. Dies hat den Vorteil, dass die Elemente dieser Tags gleichzeitig mit den Elementen der Element-Tags der Biomoleküle detektiert werden können. Denkbar ist aber auch zum Beispiel die Einführung von fluoreszierenden oder chromophoren Gruppen.

Vorzugsweise erfolgt die Identifizierung und/oder Quantifizierung der Biomoleküle und ihrer Modifikationen mittels Multielementbestimmung unter Nutzung der Tags und/oder detektierbarer, substanzspezifischer, charakteristischer Strukturmerkmale der Biomoleküle oder der modifikationserkennenden Moleküle. Die Strukturmerkmale der Biomoleküle umfassen auch Strukturmerkmale ihrer Modifikationen. Hier weist das Verfahren vorteilhafterweise eine Erweiterung des Multiplexing auf. Es können zusätzlich gleichzeitig Merkmale des Biomoleküls bzw. seiner Modifikationen bestimmt werden, für die keine zusätzliche Markierung nötig ist. In einem einzigen Detektionsschritt können so weitere Informationen gewonnen werden.

In einer Multielementbestimmung werden die Elemente der eingesetzten Element-Tags simultan detektiert. Verfügt das Biomolekül über direkt detektierbare Merkmale, die für eine bestimmte Modifikation charakteristisch sind, können diese neben den Element-Tags direkt eindeutig gemessen werden, was zur Identifizierung der entsprechenden Modifikation führt. Die Detektion und Quantifizierung der spezifischen Tags und damit der entsprechenden zugrundeliegenden Strukturen kann mit Hilfe verschiedener Techniken erfolgen. Für die Detektion können verschiedene Methoden eingesetzt werden, die eine Multielementbestimmung gestatten, wie zum Beispiel organische Massenspektrometrie, anorganische Massenspektrometrie, Atomabsorptionsspektrometrie, ortsaufgelöste Laser-Ablation in Verbindung mit ICP-MS und ortsaufgelöste Desorptions-Elektrospray-Ionisations-Massenspektrometrie. Die Wahl der Technik richtet sich nach der Art der eingesetzten Tags. Es sind auch Kombinationen aus verschiedenen Messtechniken denkbar. Die Aufzählung hat nur beispielhaften Charakter und beschränkt sich nicht auf die aufgeführten Beispiele. Für die Quantifizierung wird als "innerer Standard" jeweils der Element-Tag des zu analysierenden Biomoleküls bestimmt. Die Messergebnisse werden mit einer entsprechenden Software ausgewertet. Die Auslesung der Signale erfolgt mit verschiedenen Methoden und mit Auflösungen bis zu < 500 µm (z. B. DESI - Desorptions-Elektrospray-Ionisations-(Massen)Spektroskopie, Soft-Landing(Spray)-Desorption der Moleküle bzw. Laserdesorption in Kombination mit organischer oder anorganischer Massenspektrometrie). Die Techniken können alleine oder in Kombination eingesetzt werden.

Besonders bevorzugt unter den Multielementdetektionstechniken ist die Laserablation in Verbindung mit ICP-MS. Hier wird ein definierter Abtrag von Material aus einer Probe (zum Beispiel vom Mikroarray) mittels eines fokussierten Laserstrahles in einer Laserablationskammer genutzt. Diese ist mit einem Massenspektrometer mit einer induktiv gekoppelten Plasmaionenquelle zum Nachweis der im Probenmaterial enthaltenen Bestandteile gekoppelt. Das abgetragene Probenmaterial wird mit einem Transportgas (meist Argon) in die Plasmaionenquelle des ICP-MS transportiert und dort weiter atomisiert und ionisiert. Die LA-ICP-MS hat gegenüber anderen Multielementbestimmungsmethoden zahlreiche Vorteile. Sie ist schnell, quantitativ und auch im Spurenbereich einsetzbar. Sie gibt keinerlei Einschränkungen bezüglich der physikalischen oder chemischen Eigenschaften des Probenmaterials. Es sind nahezu alle Elemente auch quantitativ mit hoher Prazision zu bestimmen. Ihr großes Potential für die Proteinforschung ist bereits bekannt.

Ist die Modifikation ein an eine funktionelle Gruppe des Biomoleküls gebundener medizinischer Wirkstoff, zum Beispiel das Cytostatikum cis-Platin aus der Krebsbehandlung, kann das Platin direkt nachgewiesen werden. In diesem und anderen ähnlich gelagerten Beispielen kann erkannt werden, an welches Protein der Wirkstoff bzw. seine platinhaltigen Metaboliten binden. Dies gibt Hinweise auf die Wirkungsweise des Wirkstoffes.

Die Erfindung schließt nicht aus, dass das Biomolekül selbst oder die am Biomolekül befindlichen Modifikationen bereits Träger einer "natürlichen" Markierung sind. Derartige substanzspezifische Eigenstrukturen und/oder -merkmale der zu analysierenden Biomoleküle oder ihrer Modifikationen können zum Beispiel Chromophore, fluoreszierende Gruppen, bestimmte chemische Elemente oder ihre Isotope sein. Sie können gemeinsam mit elementmarkierten Modifikationen und Biomolekülen identifiziert und quantifiziert werden. Dazu zählt beispielsweise die Phosphorylierung. Der Nachweis von Phosphor spricht für eine am Protein befindliche Phosphatgruppe.

Die Bestimmung der Identität und Quantifizierung der Biomoleküle durch Multielementbestimmung erfolgt für diese Fälle in Kombination mit anderen Bestimmungsmethoden, wenn das analytisch differenzierbare Strukturmerkmal kein charakteristisches Element ist, sondern als zum Beispiel chromophore oder fluoreszierende Gruppe anderen Analysetechniken zugänglich ist. Bevorzugterweise erfolgt die Elementbestimmung und/oder -quantifizierung dann durch eine Kombination mit Fluoreszenzspektrophotometrie, UV/VIS-Spektrophotometrie oder anderen geeigneten Detektionstechniken. Die Erfindung hat den Vorteil, dass sie in einem Arbeitsgang nicht nur die Elementbestimmung gestattet, sondern auch die Bestimmung anderer charakteristischer Merkmale zulässt.

Voraussetzung für die eindeutige Identifizierung eines Biomoleküls und seiner Modifikationen ist, dass sich die an den modifikationserkennenden Molekülen detektierbaren Elemente von den Elementen des Tags des modifikationstragenden Biomoleküls unterscheiden.

Das Biomolekül kann in einer Probe vorliegen, dies einzeln oder im Gemisch. Sollen die Zusammensetzungen mehrerer unterschiedlicher biomolekülhaltiger Proben nebeneinander untersucht werden, werden die Biomoleküle jeder Probe jeweils mit unterschiedlichen Element-Tags markiert. Liegen in den Proben identische Biomoleküle vor, die an die gleiche biomolekülerkennende Spezies gebunden werden, können sie somit anhand der eingesetzten Elemente unterschieden werden. Der Nachweis des entsprechenden Elements ordnet das Biomolekül eindeutig der Herkunftsprobe zu.

Bestandteil des Verfahrens ist, dass die Modifikationen des Biomoleküls durch modifikationserkennende Moleküle erkannt werden, die mit den Modifikationen eine feste Bindung eingehen. In einer Ausgestaltung der Erfindung erkennen derartige modifikationserkennenden Moleküle am Biomolekül vorhandene funktionelle Gruppen und binden diese spezifisch. In einer bevorzugten Ausgestaltung der Erfindung erkennen modifikationserkennende Moleküle posttranslationale Modifikationen und binden diese spezifisch. Dies ist der Fall, wenn es sich bei dem Biomolekül um ein Protein handelt. Das erfindungsgemäße Verfahren erweitert damit deutlich die Möglichkeiten in der Proteomanalyse.

In einer weiteren Ausgestaltung der Erfindung erkennen und binden modifikationserkennende Moleküle spezifisch an das Biomolekül gebundene Moleküle, Atome oder Atomgruppen. Dabei kann es sich zum Beispiel um Adduktbildungen des Biomoleküls mit Pharmaka, Toxinen, Metabolismusprodukten des Stoffwechsels, Nahrungsmittelbestandteilen oder deren Abbauprodukten und mit Allergenen handeln.

In bevorzugten Ausgestaltungen der Erfindung sind die biomolekülerkennende Spezies und das modifikationserkennende Molekül ebenfalls ein Biomolekül. Derartige Biomoleküle sind bereits in großer Zahl beschrieben worden. Sie sind oft kommerziell erhältlich, oder es sind für den Fachmann leicht nachvollziehbare Verfahren zu ihrer Herstellung beschrieben worden. In der Proteinanalyse handelt es sich bei den biomolekülerkennenden Spezies um Antikörper, bei den modifikationserkennenden Molekülen kann es sich um Lektine, anti-Ubiquitin oder anti-SUMO handeln.

Nach der Probenvorbereitung, die sich nach der Art der Probe richtet und vom Fachmann unter Kenntnis der entsprechenden Literatur leicht durchgeführt werden kann, werden die Biomoleküle mit ausgewählten Element-Tags markiert. Diese Art der Markierung hat den Vorteil, dass als Elemente fast alle Elemente des Periodensystems in Frage kommen, ausgenommen Wasserstoff und die Edelgase. Weiterhin von Vorteil ist, dass sich im bevorzugten Fall der Proteinmarkierung viele funktionelle Gruppen innerhalb der Polypeptidkette anbieten, an die ein Element-Tag leicht binden kann (zum Beispiel Hydroxylgruppen, Aminogruppen, Thiolgruppen). Derartige Markierungen sind bereits offenbart. Oft kann auf kommerziell erhältliche Element-Tags zurückgegriffen werden. Sie können auch leicht nach bekannten Methoden hergestellt werden.

Es ist von Vorteil, wenn die für die Markierung der Biomoleküle und/oder der modifikationserkennenden Moleküle eingesetzten Elemente der Elementtags Metalle sind. Es können aber auch alle anderen Elemente des Periodensystems und ihre Isotope verwendet werden, ausgenommen Wasserstoff und Edelgase. Die meisten Metalle sind in der Regel nicht in Biomolekülen oder ihren Modifikationen enthalten, so dass ihr Nachweis bei der Detektion ein eindeutiger Hinweis auf ihre Herkunft aus dem Element-Tag ist. Metalle sind der Multielementdetektion leicht zugänglich. Sie sind einfach zu chelatisieren und über funktionalisierte Chelate gezielt fest an funktionelle Gruppen des Biomoleküls zu binden. Diese Markierungen gehören zum Stand der Technik. Die Tags verändern das Biomolekül und seine Modifikationen selbst nicht.

Besonders bevorzugt sind Lanthanide, für die zahlreiche stabile Chelatkomplexe beschrieben sind, die als Element-Tag geeignet sind. In einer besonders bevorzugten Ausgestaltung der Erfindung besteht der Chelatkomplex aus einem Lanthanidmetall und einem Liganden vom Typ DTPA, DTPA-Derivat, DOTA oder DOTA-Derivat. Für diese Komplexe sind in freier oder gebundener Form Stabilitätskonstanten für verschiedene Metalle beschrieben worden. Diese Konstanten belegen, dass Metallaustauschreaktionen mit anderen Metall-Chelat-Komplexen nicht messbar erfolgen. Wird folglich das zur Markierung des Biomoleküls verwendete Metall detektiert, ist dies ein eindeutiger Beweis für das Vorhandensein eines Biomoleküls, das gezielt mit diesem Komplex markiert wurde.

Das erfindungsgemäße Verfahren findet Verwendung zur Genomanalyse, zur Metabolitenanalyse, in der Rezeptorforschung, zum Drogen-Screening, in der Cell-onthe-Chip-Technologie, zur Computer-Simulation von zellulären Vorgängen, in der Systembiologie, zur Untersuchung von bimolekularen Interaktionen und der endokrinen Disruption (Modulation hormoneller Rezeptoren durch Umweltgifte), in der Toxikologie, in der Umweltanalytik, in der Pharmakologie, zur Entwicklung neuer Arzneimittel, zur medizinischen Diagnostik, zur Vorsorge und zum Screening in der Krebsdiagnostik, zur Untersuchung einer von der Pflanze, dem Tier oder dem Menschen entnommenen und Biomoleküle enthaltenden Probe und allgemein in der biochemischen Forschung.

Ein weiterer Aspekt der Erfindung sind das Design und die Zusammenstellung kompatibler Detektionsreagenzien, zum Beispiel in Form eines Kits, das in dem erfindungsgemäßen Verfahren zur Multiplexbestimmung von Biomolekülen und ihrer Modifikationen verwendet werden kann. Eine Ausgestaltung der Erfindung sieht vor, für ausgewählte Anwendungen ein derartiges Kit bereitzustellen. Es kann beispielsweise zur Bestimmung des Glykolysierungsgrades eines bestimmten Proteins dienen. Dieses in dem erfindungsgemäßen Verfahren verwendbare diagnostische Kit zur Identifizierung und Quantifizierung von Proteinen enthält die im zugehörigen unabhängigen Anspruch aufgeführten Komponenten

In einer weiteren Ausgestaltung der Erfindung umfasst das diagnostische Kit ferner einen Linker zur Fixierung der proteinerkennenden Spezies auf dem Mikroarray.
(b) Markierungsreagenzien zur Markierung von Biomolekülen
(c) modifikationserkennende Spezies und Markierungsreagenzien zur Markierung der modifikationserkennenden Spezies oder bereits markierte modifikationserkennende Spezies.

Bei den Markierungsreagenzien zur Markierung der Biomoleküle handelt es sich um Element-Tags.

In einer weiteren Ausgestaltung der Erfindung umfasst das diagnostische Kit ferner einen Linker zur Fixierung der biomolekülerkennenden Spezies auf dem Mikroarray.

Besonders bevorzugt ist die Verwendung des diagnostischen Kits zur Proteinanalyse.

Außerdem denkbar ist die Verwendung des diagnostischen Kits zur Genom- und Metabolitenanalyse, zur Rezeptorforschung, zum Drogen-Screening, zur "Cell on the Chip"-Technologie, zur Computer-Simulation von zellulären Vorgängen, in der Systembiologie, zur Untersuchung bimolekularer Interaktionen und der endokrinen Disruption (Modulation hormoneller Rezeptoren durch Umweltgifte), in der Toxikologie, in der Umweltanalytik, in der Pharmakologie, zur Entwicklung neuer Arzneimittel, zur medizinischen Diagnostik, zur Vorsorge und zum Screening in der Krebsdiagnostik, zur Untersuchung einer von der Pflanze, dem Tier oder dem Menschen entnommenen und Biomoleküle enthaltenden Probe und allgemein in der biochemischen Forschung.

Es wurde ein analytisches Verfahren entwickelt wurde, welches Biomoleküle und seine Modifikationen simultan identifiziert, quantifiziert und charakterisiert und welches im Hochdurchsatzverfahren einsetzbar ist. Dies ist gleichzeitig für zahlreiche Proben, Probengemische und deren Bestandteile möglich.

Es ist im Mikromaßstab durchführbar und tauglich für den Mikroarray-Maßstab. Es werden komplexe Analysendaten mit hohem Informationsgehalt hinsichtlich vorhandener Biomoleküle und abgewandelter Biomolekülstrukturen (Modifikationen) zur Verfügung gestellt. Grundlage der Erfindung sind Mikroarrays mit speziell definierten Bindungsstrukturen für zu analysierende Moleküle. Neu sind spezielle Kombinationen ausgewählter, mit differenzierenden Tags versehener Erkennungsmoleküle für ein Multiplexing, das eine simultane Identifizierung und Quantifizierung von Biomolekülen und ihrer verschiedenen Modifikationen erlaubt.

Ausgestaltungen der Erfindung sind Gegenstand der Unteransprüche. Ausführungsbeispiele sind nachstehend unter Bezugnahme auf die beigefügten Zeichnungen näher erläutert.

### Kurze Beschreibung der Zeichnungen

- Fig. 1: zeigt schematisch den Schritt der Elementmarkierung von identischen Biomolekülen.
- Fig. 2: zeigt schematisch den Schritt der Elementmarkierung von Biomolekülen eines Biomolekülgemisches.
- Fig. 3: zeigt schematisch den Schritt der Bindung der markierten Biomoleküle aus Fig. 1 an zugehörige biomolekülerkennende Spezies, die auf einer Oberfläche immobilisiert sind.
- Fig. 4: zeigt schematisch den Schritt der Bindung der markierten Biomoleküle des Biomolekülgemisches aus Fig. 2 an die jeweils zugehörigen biomolekülerkennenden Spezies, die auf einer Oberfläche immobilisiert sind.
- Fig. 5: zeigt schematisch den Schritt der Bindung von markierten molekülerkennenden Molekülen eines Typs an die zugehörigen Modifikationen der entsprechenden Biomoleküle.
- Fig. 6: zeigt schematisch den Schritt der Bindung von unterschiedlich markierten molekülerkennenden Molekülen verschiedenen Typs an die zugehörigen Modifikationen der entsprechenden Biomoleküle.
- Fig. 7: zeigt in einer schematischen Gegenüberstellung zwei Beispiele für ein Protein, das Träger verschiedener posttranslationaler Modifikationen ist, und die zugehörigen nach dem erfindungsgemäßen Verfahren vorgenommenen Markierungen.
- Fig. 8: zeigt ein Übersichtsschema, das die Wechselwirkungen zwischen Benzol, Benzolmetaboliten sowie reaktiven Sauerstoffspezies (ROS) mit Signaltransduktionswegen und Enzymen, die zu benzolinduzierten Modifikationen regulatorischer Proteine in Knochenmarkszellen führen, illustriert.

### Beschreibung der Ausführungsbeispiele

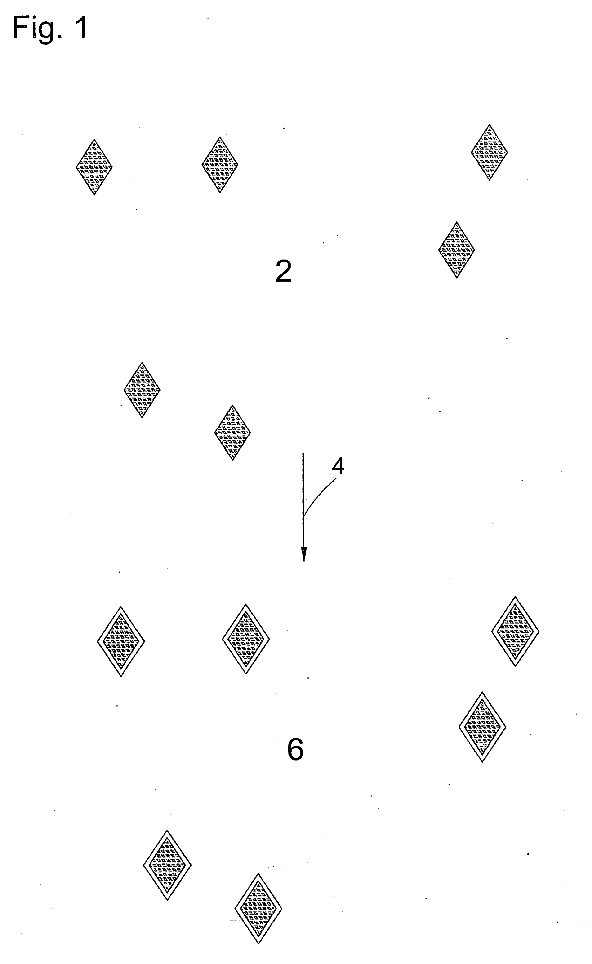

In Figur 1 ist eine Biomoleküllösung 2 gezeigt, die nur eine Art von Biomolekülen (angedeutet durch schwarze Rauten) enthält. Diese wird in einem Markierungsschritt 4 mit Element-Tags gleichen Typs versetzt. Diese Element-Tags gehen mit den Biomolekülen eine feste Verbindung ein und bilden so eine Lösung aus markierten Biomolekülen 6. Die Markierung wird durch die graue Umrandung der die Biomoleküle darstellenden Rauten angedeutet.

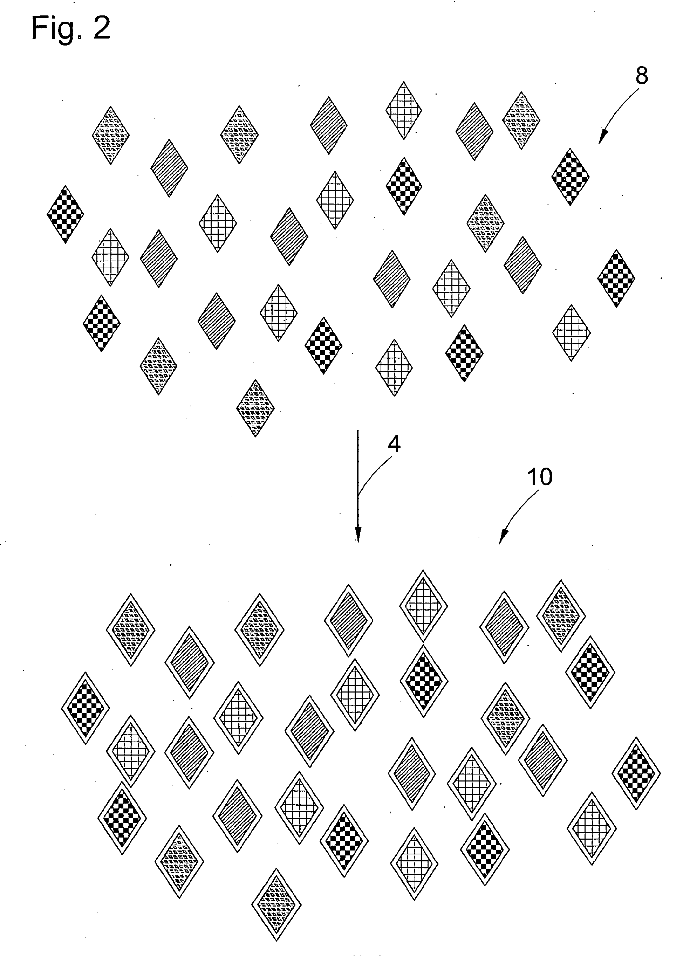

In Figur 2 wird das System aus Figur 1 dahingehend erweitert, dass mehrere Arten von Biomolekülen in einer Biomolekülmischung 8 (angedeutet durch verschieden gemusterte Rauten) vorliegen. Diese werden ebenfalls in einem Markierungsschritt 4 mit Element-Tags gleichen Typs versetzt. Diese Element-Tags gehen mit den Biomolekülen eine feste Verbindung ein und bilden so eine Lösung aus einem Gemisch der so markierten Biomoleküle 10. Die Markierung wird durch die graue Umrandung der die Biomoleküle darstellenden Rauten angedeutet.

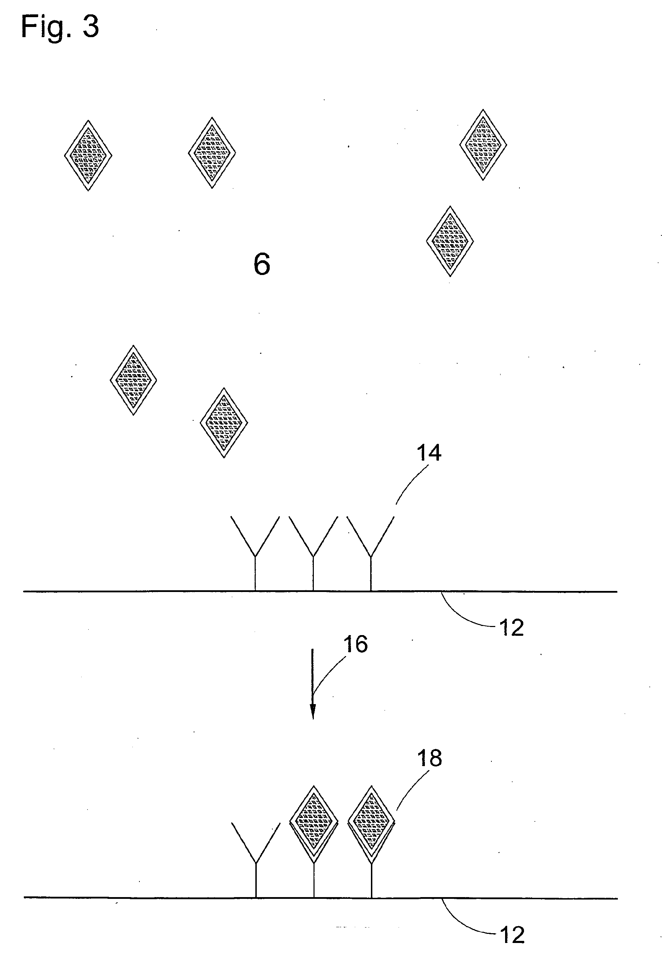

In Figur 3 werden die markierten Biomoleküle 6 aus Figur 1 in einem Schritt 16 mit auf einer Oberfläche 12 immobilisierten biomolekülerkennenden Spezies 14 kontaktiert. Die biomolekülerkennenden Spezies 14 sind spezifisch für die Biomoleküle aus der Biomoleküllösung 2 und binden diese. Es bildet sich ein Komplex 18 aus markierten Biomolekülen 6 und auf der Oberfläche immobilisierten biomolekülerkennenden Spezies 14.

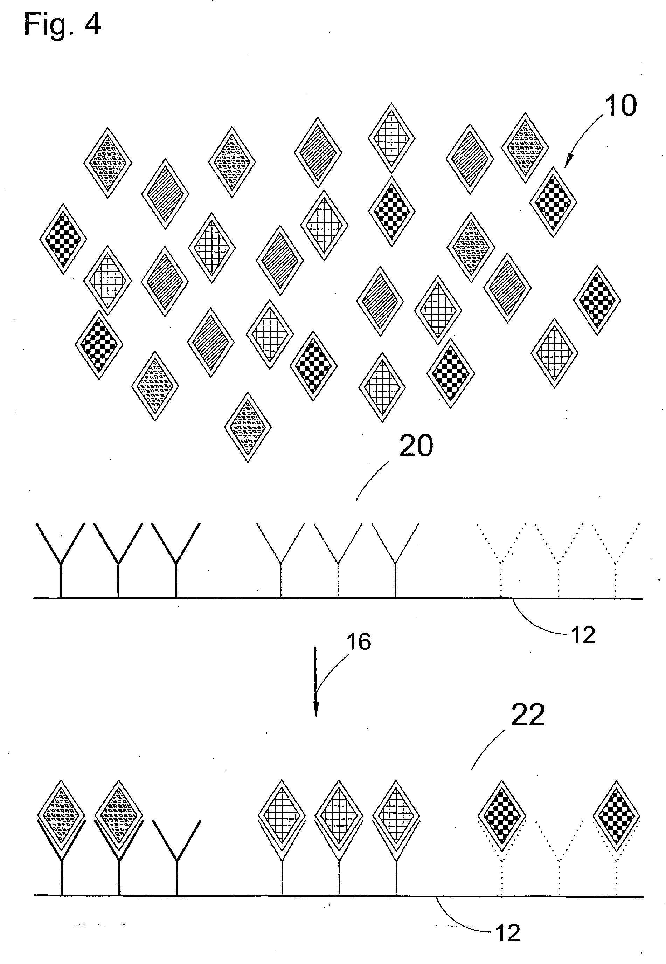

In Figur 4 wird das markierte Biomolekülgemisch 10 aus Figur 2 in einem Schritt 16 mit auf einer Oberfläche 12 immobilisierten biomolekülerkennenden Spezies 20 kontaktiert. Hier sind verschiedene Arten von biomolekülerkennenden Spezies auf der Oberfläche verankert worden. Diese unterschiedlichen biomolekülerkennenden Spezies 20 sind jeweils spezifisch für bestimmte Biomoleküle aus der Biomolekülgemischlösung 8 und binden das jeweils zugehörige Biomolekül. Es bilden sich Komplexe 22 aus den verschiedenen markierten Biomolekülen 10 und den zugehörigen auf der Oberfläche 12 immobilisierten biomolekülerkennenden Spezies 22 verschiedenen Typs.

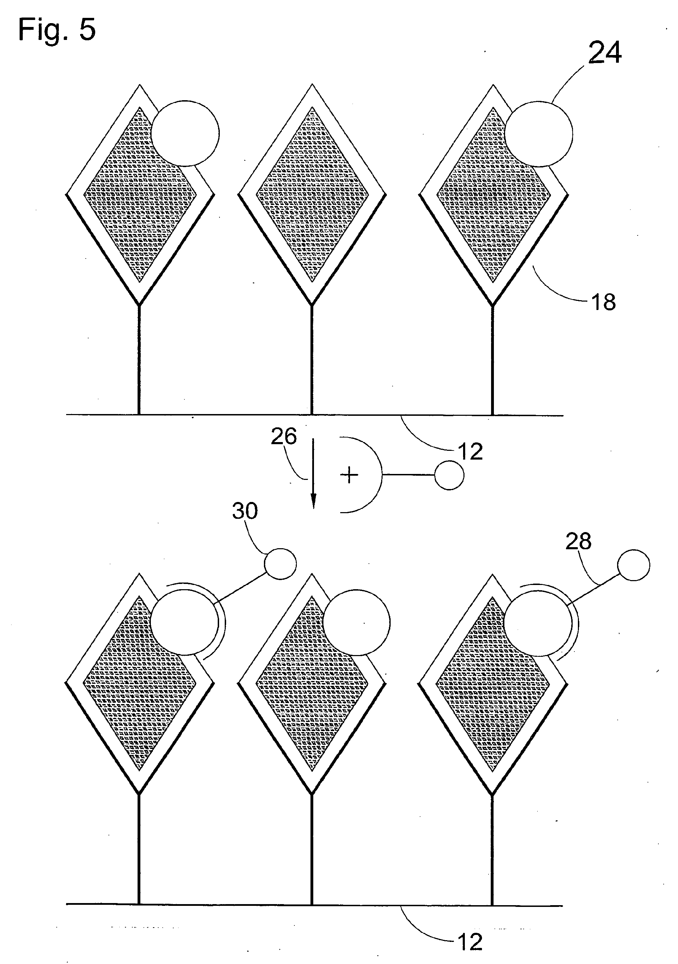

In Figur 5 ist gezeigt, dass die entsprechend Figur 3 in einem Komplex 18 an die immobilisierten biomolekülerkennenden Spezies gebundenen markierten Biomoleküle Träger einer Modifikation 24 sind. In einem weiteren Markierungsschritt 26 werden diese Komplexe mit anders als das Biomolekül markierten modifikationserkennenden Molekülen, die aus dem modifikationserkennenden Molekül 28 und seiner Markierung 30 bestehen, kontaktiert. Das modifikationserkennende Molekül bindet an die zugehörige Modifikation. Es bildet sich somit ein auf der Oberfläche verankertes System, das aus biomolekülerkennender Spezies, markiertem Biomolekül mit Modifikation und daran gebundenem markiertem modifikationserkennendem Molekül besteht. Mit einer geeigneten Detektionsmethode können nun die verschiedenen Markierungen identifiziert und quantifiziert werden. Damit werden indirekt das Biomolekül und seine Modifikation identifiziert und quantifiziert.

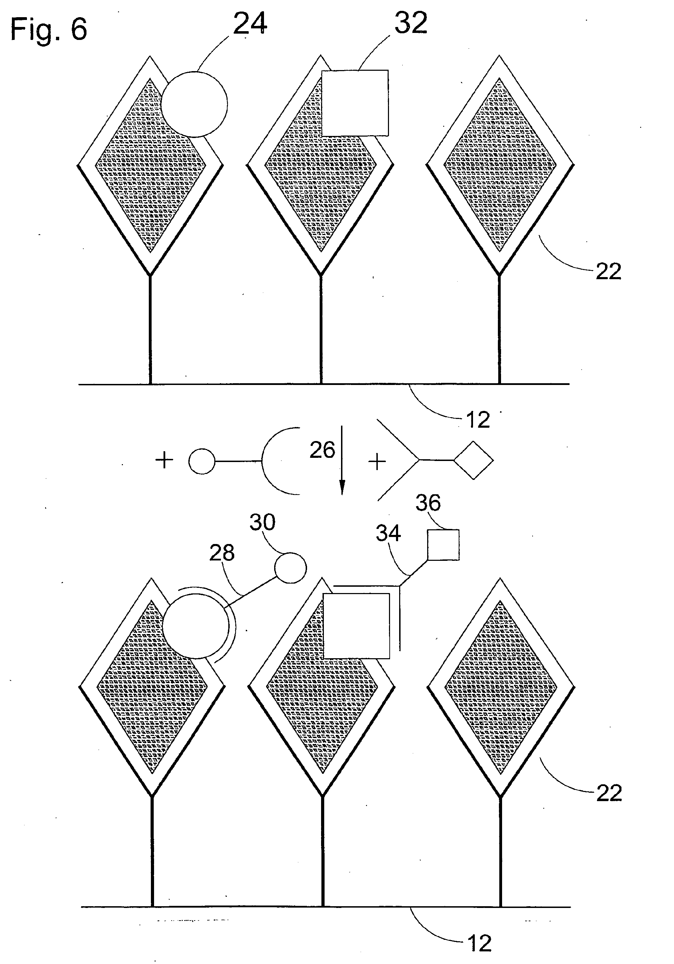

In Figur 6 ist gezeigt, dass die entsprechend Figur 4 in einem Komplex 22 an die immobilisierten biomolekülerkennenden Spezies gebundenen markierten Biomoleküle eines Typs sich untereinander in ihren Modifikationen 24, 32 unterscheiden. Hier wurde nur ein Biomolekültyp aus dem ursprünglichen Gemisch herausgegriffen, der exemplarisch auch für die anderen Biomoleküle der Mischung gilt. In einem weiteren Markierungsschritt 26 werden diese Komplexe mit anders als das Biomolekül markierten modifikationserkennenden Molekülen, die aus dem modifikationserkennenden Molekül 28 bzw. 34 und seiner Markierung 30 bzw. 36 bestehen, kontaktiert. Jedes modifikationserkennende Molekül bindet an die jeweils zugehörige Modifikation 24 bzw. 32. Es bildet sich somit ein auf der Oberfläche 12 verankertes System, das aus biomolekülerkennender Spezies, markiertem Biomolekül mit jeweiliger Modifikation und daran gebundenem entsprechendem markiertem modifikationserkennendem Molekül besteht. Mit einer geeigneten Detektionsmethode können nun die verschiedenen Markierungen identifiziert und quantifiziert werden. Damit werden indirekt das Biomolekül und seine unterschiedlichen Modifikationen identifiziert und quantifiziert.

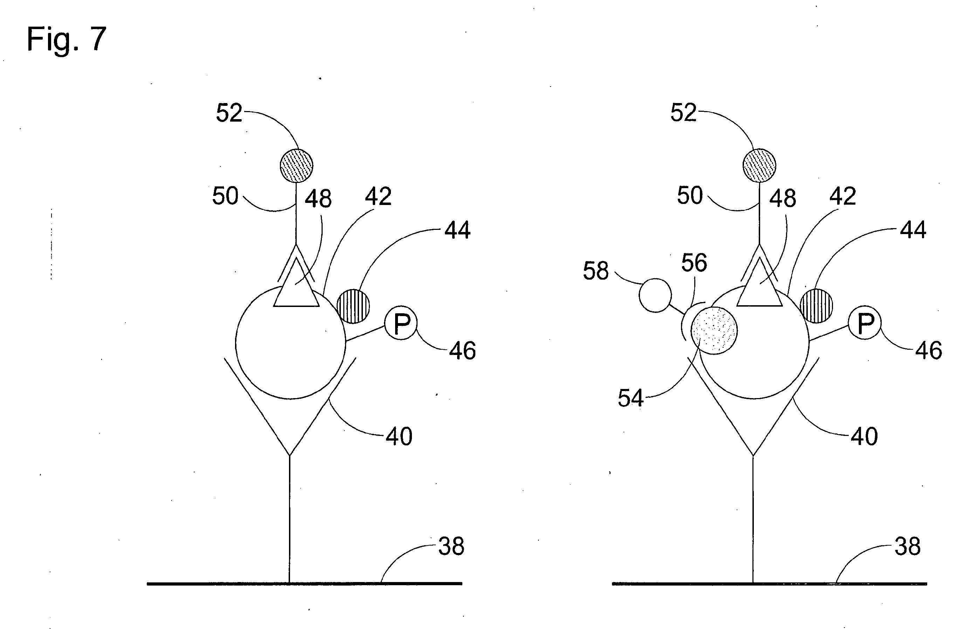

In einem konkreten Ausführungsbeispiel sind in Figur 7 zwei mögliche Versionen von nach dem erfindungsgemäßen Verfahren behandelten Biomolekülen dargestellt. Bei dem mit 42 bezeichneten Biomolekül handelt sich in beiden Fällen um das gleiche Protein p53. Im linken Fall ist das Protein 42 Träger einer Phosphatgruppe 46 aus einer posttranslationalen Phosphorylierung und Träger einer Zuckerfunktion 48 aus einer posttranslationalen Glykosylierung. Im rechten Fall trägt das Protein 42 zusätzlich eine SUMO-Gruppe 54. Das Protein 42 wird in beiden Fällen zunächst mit einem Eu-DOTA-Chelatkomplex 44 markiert. Auf einer Gold-Mikroarrayoberfläche 38 wird die biomolekülerkennende Spezies 40 in Form des Antikörpers anti-p53 immobilisiert. Der Array wird mit der Lösung der markierten p53-Proteine in Kontakt gebracht. Die Antikörper anti-p53 gehen mit den europiummarkierten p53-Molekülen feste Bindungen ein. Dann werden modifikationserkennende Moleküle 50, 56 mit den Lanthanid-DOTA-Komplexen 52, 58 markiert. Für die Modifikation in Form einer Zuckergruppe 48 wird als modifikationserkennendes Molekül 50 ein mit einem Gd-DOTA-Chelatkomplex 52 markiertes geeignetes Lektin eingesetzt. Für die Modifikation in Form einer SUMO-Gruppe 54 wird als modifikationserkennendes Molekül 56 ein mit einem Sm-DOTA-Chelatkomplex 58 markierter Antikörper vom Typ anti-SUMO eingesetzt. Die modifikationserkennenden Moleküle 50, 56 werden mit den immobilisierten markierten p53-Proteinen kontaktiert. Die entsprechenden Modifikationen 48, 54 binden die zugehörigen modifikationserkennenden Moleküle 50, 56. Es bilden sich Systeme aus Protein p53 40 mit entsprechenden Modifikationen, Antikörper anti-p53 und an die Modifikationen gebundenen spezifischen modifikationserkennenden Spezies. Im linken Fall ist das Protein Träger von zwei Modifikationen, im rechten Fall Träger von drei Modifikationen. Das erfindungsgemäße Verfahren lässt die Identifizierung und Quantifizierung von mehreren verschiedenen Modifikationen zu. Der Mikroarray wird in eine Laserablationszelle überführt. Das Proteinmaterial wird mit Hilfe eines Laserstrahls abgetragen und mit einem Transportgas in ein ICP-MS-Gerät überführt. Dort werden die verschiedenen zur Markierung eingesetzten Lanthanide der einzelnen Punkte des Mikroarrays vermessen. Gleichzeitig wird der in der Phosphatgruppe enthaltene Phosphor vermessen. Dies ist ein Beispiel für ein charakteristisches Strukturmerkmal einer Modifikation, die direkt bestimmt werden kann, ohne dass seine Markierung nötig ist. Der Antikörper 40 und die modifikationserkennenden Moleküle 50, 56 müssen dephosphoryliert vorliegen bzw. vor ihrem Einsatz gegebenenfalls mit Proteinphosphatasen dephosphoryliert werden, damit das Phosphorsignal in der Detektion eindeutig dem Protein 42 zugeordnet werden kann.

Die qualitative und quantitative Bestimmung der Elemente Eu, Gd, Sm und P mittels LA-ICP-MS gestattet in diesem Multiplexing-Ansatz die gleichzeitige Identifizierung und Quantifizierung des Proteins 42 und seiner jeweiligen Modifikationen 46, 48 und im rechten Fall der Figur 7 auch 54.

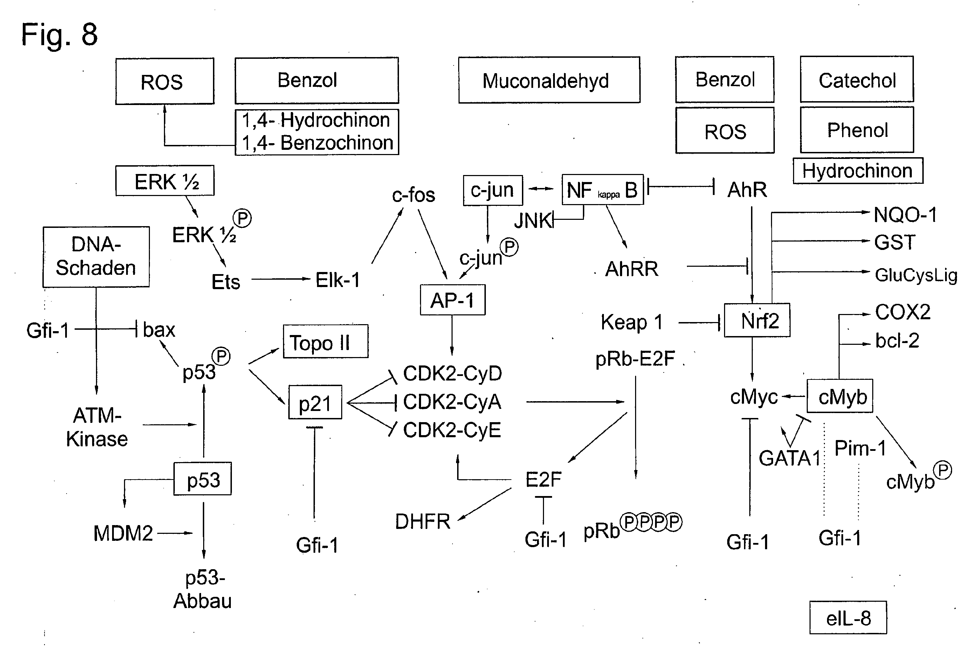

In Figur 8 (die kleinen Kreise symbolisieren Phosphorylierungen, die den Aktivitätszustand der Proteine verändern) soll verdeutlicht werden, dass das erfindungsgemäße Verfahren ein hohes Verwendungspotential in der Toxikologie, insbesondere in der Krebsforschung, hat. Benzol hat eine knochenmarktoxische Wirkung. Die Ursache dieser Wirkung ist die Bildung reaktiver Metaboliten, die kovalent an DNA, RNA und Proteine binden. Oxidationsprodukte des Benzols entstehen in der Leber und zum Teil auch im Knochenmark selber. Wichtige reaktive Metaboliten sind Benzolepoxid, Muconaldehyd, Phenol, Hydrochinon und Catechol. Der Eingriff dieser und anderer Metaboliten in Signaltransduktionswege und ihre Wechselwirkungen mit Enzymen führen zu Proteinmodifikationen von Proteinen, die an wichtigen Schaltstellen der Regulation von Zellteilung und Zelldifferenzierung stehen. Diese Proteine und ihre Modifikationen können mit dem erfindungsgemäßen Verfahren simultan erfasst werden.

Neben der dargestellten Phosphorylierung von Proteinen kommen weitere Modifikationen wie zum Beispiel Ubiquitinylierung und Sumoylierung in Betracht. Der Nachweis dieser Modifikationen kann wie in Fig. 7 gezeigt erfolgen.

## Patentansprüche

1. Verfahren zur simultanen Bestimmung der Identität und/oder Quantifizierung von Biomolekülen und mindestens einer ihrer Modifikationen, mit den folgenden Schritten:
(a) Immobilisierung von biomolekülerkennenden, das Biomolekül spezifisch bindenden Spezies an definierten Stellen auf einer Oberfläche;
(b) Markieren der Biomoleküle mit einem Element-Tag;
(c) Kontaktieren der Biomoleküle mit der die biomolekülerkennenden Spezies tragenden Oberfläche;
(d) Kontaktieren der an die biomolkülerkennenden Spezies gebundenen Biomoleküle mit modifikationserkennenden Molekülen, welche analytisch differenzierbar sind, wobei das modifikationserkennende Molekül selektiv nur eine bestimmte Modifikation des Biomoleküls erkennt und mit dieser eine feste kovalente oder nichtkovalente Bindung eingeht und die Modifikationen des Biomoleküls ausgewählt sind aus seinen funktionellen Gruppen und aus mit ihm verknüpften Atomen, Atomgruppen oder Molekülen.

2. Verfahren nach Anspruch 1, **dadurch gekennzeichnet, dass** auf der Oberfläche eine Vielzahl von biomolekülerkennenden Spezies immobilisiert werden kann.

3. Verfahren nach Anspruch 1 oder 2, **dadurch gekennzeichnet, dass** die modifikationserkennenden Spezies vor dem Kontaktieren mit den Biomolekülen mit spezifischen, analytisch differenzierbaren Tags markiert werden.

4. Verfahren nach einem der Ansprüche 1 bis 3, **dadurch gekennzeichnet, dass** die Identifizierung und/oder Quantifizierung der Biomoleküle und ihrer Modifikationen mittels Multielementbestimmung erfolgt.

5. Verfahren nach Anspruch 4, **dadurch gekennzeichnet, dass** die Elementbestimmung
durch eine Kombination mit Fluoreszenzspektrophotometrie und/oder UV/VIS-Spektrophotometrie erfolgt.

6. Verfahren nach einem der vorgehenden Ansprüche, **dadurch gekennzeichnet, dass** das Biomolekül in einer Probe vorliegt und Biomolekülgemische verschiedener Proben jeweils mit unterschiedlichen Element-Tags markiert werden.

7. Verfahren nach einem der vorhergehenden Ansprüche, **dadurch gekennzeichnet, dass** die modifikationserkennenden Moleküle am Biomolekül vorhandene funktionelle Gruppen erkennen und spezifisch binden.

8. Verfahren nach einem der vorhergehenden Ansprüche, **dadurch gekennzeichnet, dass** die modifikationserkennenden Moleküle posttranslationale Modifikationen erkennen und spezifisch an sie binden.

9. Verfahren nach einem der vorhergehenden Ansprüche, **dadurch gekennzeichnet, dass** die modifikationserkennenden Moleküle an das Biomolekül gebundene Moleküle, Atome oder Atomgruppen erkennen und spezifisch binden.

10. Verfahren nach einem der vorhergehenden Ansprüche **dadurch gekennzeichnet, dass** die Oberfläche eine Mikroarray-Oberfläche ist.

11. Verfahren nach Anspruch 10, **dadurch gekennzeichnet, dass** die Oberfläche des eingesetzten Mikroarrays aus Metallclustern, Metallen, organischen oder anorganischen Polymeren, Glas oder Halbleitern besteht.

12. Verfahren nach Anspruch 11, **dadurch gekennzeichnet, dass** die Oberfläche des eingesetzten Mikroarrays eine Goldoberfläche ist.

13. Verfahren nach einem der Ansprüche 10-12, **dadurch gekennzeichnet, dass** die biomolekülerkennenden Spezies mit Linkern auf der Oberfläche des Mikroarrays gebunden werden.

14. Verfahren nach Anspruch 13, **dadurch gekennzeichnet, dass** die Linker thiolgruppenfunktionalisierte Linker sind.

15. Verfahren nach einem der vorhergehenden Ansprüche 3 bis 14, **dadurch gekennzeichnet, dass** die für die Markierung der Biomoleküle und/oder der modifikationserkennenden Moleküle eingesetzten Elemente der Elementtags Metalle sind.

16. Verfahren nach Anspruch 15, **dadurch gekennzeichnet, dass** das für die Markierung der Biomoleküle und/oder der modifikationserkennenden Moleküle eingesetzte Metall ein Lanthanid ist.

17. Verfahren nach einem der vorhergehenden Ansprüche 2 bis 16, **dadurch gekennzeichnet, dass** der Element-Tag ein Chelatkomplex ist.

18. Verfahren nach Anspruch 17, **dadurch gekennzeichnet, dass** der Chelatkomplex aus einem Lanthanidmetall und einem Liganden vom Typ DTPA, DTPA-Derivat, DOTA öder DOTA-Derivat besteht.

19. Verfahren nach einem der vorhergehenden Ansprüche, **dadurch gekennzeichnet, dass** die biomolekülerkennende Spezies ebenfalls ein Biomolekül ist.

20. Verfahren nach einem der vorhergehenden Ansprüche, **dadurch gekennzeichnet, dass** das modifikationserkennende Molekül ebenfalls ein Biomolekül ist.

21. Diagnostisches Kit zur Identifizierung und Quantifizierung von Proteinen nach einem Verfahren der Ansprüche 1 bis 20, enthaltend
(a) ein mit proteinerkennenden Spezies versehenes Mikroarray oder ein Mikroarray und auf dem Mikroarray zu fixierende proteinerkennende Spezies,
(b) Markierungsreagenzien zur Markierung von Proteinen,
(c) Spezies, die posttranslationale Modifikationen erkennen, und Markierungsreagenzien zur Markierung der Spezies, die posttranslationale Modifikationen erkennen, oder bereits markierte Spezies, die posttranslationale Modifikationen erkennen, **dadurch gekennzeichnet, dass**
es sich bei den Markierungsreagenzien jeweils um Element-Tags handelt.

22. Diagnostisches Kit nach Anspruch 21, **dadurch gekennzeichnet, dass** es einen Linker zur Fixierung der proteinerkennenden Spezies enthält.

23. Verwendung des diagnostischen Kits nach Anspruch 21 oder 22 zur Proteinanalyse.

## Claims

1. A method for simultaneously determining the identity of and/or for quantifying biomolecules and at least one of their modifications, having the following steps:
(a) immobilizing biomolecule-recognizing species which specifically bind the biomolecule at predetermined sites on a surface;
(b) tagging the biomolecules with an element tag;
(c) contacting the biomolecules with the surface carrying the biomolecule-recognizing species;
(d) contacting the biomolecules bound to the biomolecule-recognizing species with modification-recognizing molecules which can be differentiated analytically, wherein the modification-recognizing molecule selectively recognizes only one specific modification of the biomolecule and forms a strong covalent or noncovalent bond therewith, and the modifications of the biomolecule are selected from its functional groups and from atoms, groups of atoms or molecules associated therewith.

2. The method according to claim 1, **characterized in that** a plurality of biomolecule-recognizing species can be immobilized on the surface.

3. The method according to claim 1 or claim 2, **characterized in that** the modification-recognizing species are tagged with specific tags which can be differentiated analytically prior to contacting with the biomolecules.

4. The method according to one of claims 1 to 3, **characterized in that** identification and/or quantification of the biomolecules and their modifications is carried out by means of multi-element determination.

5. The method according to claim 4, **characterized in that** the element determination is carried out by means of a combination with fluorescence spectrophotometry and/or UV/VIS spectrophotometry.

6. The method according to one of the preceding claims, **characterized in that** the biomolecule is present in a sample and biomolecule mixtures of various samples are each tagged with different element tags.

7. The method according to one of the preceding claims, **characterized in that** the modification-recognizing molecules recognize and specifically bind functional groups present on the biomolecule.

8. The method according to one of the preceding claims, **characterized in that** the modification-recognizing molecules recognize and specifically bind to post-translational modifications.

9. The method according to one of the preceding claims, **characterized in that** the modification-recognizing molecules recognize and specifically bind to molecules, atoms or groups of atoms bound to the biomolecule.

10. The method according to one of the preceding claims, **characterized in that** the surface is a microarray surface.

11. The method according to claim 10, **characterized in that** the surface of the microarray employed consists of metal clusters, metals, organic or inorganic polymers, glass or semiconductors.

12. The method according to claim 11, **characterized in that** the surface of the microarray employed is a gold surface.

13. The method according to one of claims 10 - 12, **characterized in that** the biomolecule-recognizing species is bound to the surface of the microarray via linkers.

14. The method according to claim 13, **characterized in that** the linkers are thiol group-functionalized linkers.

15. The method according to one of the preceding claims 3 to 14, **characterized in that** the elements of the element tag used to tag the biomolecules and/or the modification-recognizing molecules are metals.

16. The method according to claim 15, **characterized in that** the metal used to tag the biomolecules and/or the modification-recognizing molecules is a lanthanide.

17. The method according to one of the preceding claims 2 to 16, **characterized in that** the element tag is a chelate complex.

18. The method according to claim 17, **characterized in that** the chelate complex consists of a lanthanide metal and a ligand of the DTPA, DTPA derivative, DOTA or DOTA derivative type.

19. The method according to one of the preceding claims, **characterized in that** the biomolecule-recognizing species is also a biomolecule.

20. The method according to one of the preceding claims, **characterized in that** the modification-recognizing molecule is also a biomolecule.

21. A diagnostic kit for identifying and quantifying proteins in accordance with a method of claims 1 to 20, containing:
(a) a microarray provided with protein-recognizing species or a microarray and protein-recognizing species to be fixed on the microarray;
(b) tagging reagents to tag the proteins;
(c) species which recognize post-translational modifications, and tagging reagents to tag the species which recognize post-translational modifications, or ready-tagged species which recognize post-translational modifications, **characterized in that**
each tagging reagent is an element tag.

22. A diagnostic kit according to claim 21, **characterized in that** it contains a linker for fixing the protein-recognizing species.

23. Use of the diagnostic kit according to claim 21 or 22, for protein analysis.

## Revendications

1. Procédé pour l'identification et/ou la quantification simultanée de biomolécules et d'au moins une de leurs modifications, comprenant les étapes suivantes consistant à :
(a) immobiliser des espèces identifiant les biomolécules et se liant spécifiquement aux biomolécules à des endroits définis sur une surface ;
(b) marquer les biomolécules avec un tag ;
(c) mettre en contact les biomolécules avec la surface porteuse des espèces identifiant les biomolécules ;
(d) mettre en contact les biomolécules liées aux espèces identifiant les biomolécules avec des molécules identifiant les modifications et différenciables par l'analyse, la molécule qui identifie les modifications n'identifiant sélectivement qu'une modification déterminée de la biomolécule et formant avec celle-ci une liaison fixe covalente ou non covalente et les modifications de la biomolécule étant choisies parmi ses groupes fonctionnels et parmi les atomes, groupes d'atomes ou molécules liés à celle-ci.

2. Procédé selon la revendication 1, **caractérisé en ce qu'**une multitude d'espèces identifiant les biomolécules peut être immobilisée sur la surface.

3. Procédé selon la revendication 1 ou 2, **caractérisé en ce que** les espèces identifiant les modifications sont marquées par des tags spécifiques, différenciables par l'analyse, avant la mise en contact avec les biomolécules.

4. Procédé selon l'une des revendications 1 à 3, **caractérisé en ce que** l'identification et/ou la quantification des biomolécules et de leurs modifications s'effectue(nt) au moyen d'une détermination multiéléments.

5. Procédé selon la revendication 4, **caractérisé en ce que** la détermination des éléments s'effectue par une combinaison de la spectrométrie de fluorescence et/ou la spectrophotométrie UV/VIS.

6. Procédé selon l'une des revendications précédentes, **caractérisé en ce que** la biomolécule est présente dans un échantillon et que les mélanges de biomolécules de divers échantillons sont marqués respectivement avec différents tags.

7. Procédé selon l'une des revendications précédentes, **caractérisé en ce que** les molécules identifiant les modifications identifient les groupes fonctionnels présents sur la biomolécule et les lient spécifiquement.

8. Procédé selon l'une des revendications précédentes, **caractérisé en ce que** les molécules identifiant les modifications identifient les modifications post-traductionnelles et les lient spécifiquement.

9. Procédé selon l'une des revendications précédentes, **caractérisé en ce que** les molécules identifiant les modifications identifient les molécules, atomes ou groupes d'atomes liés à la biomolécule et les lient spécifiquement.

10. Procédé selon l'une des revendications précédentes, **caractérisé en ce que** la surface est une surface à micropuces.

11. Procédé selon la revendication 10, **caractérisé en ce que** la surface de la micropuce utilisée est constituée de clusters métalliques, de métaux, de polymères organiques ou minéraux, de verre ou de semi-conducteurs.

12. Procédé selon la revendication 11, **caractérisé en ce que** la surface de la micropuce utilisée est une surface en or.

13. Procédé selon l'une des revendications 10 à 12, **caractérisé en ce que** les espèces identifiant les biomolécules sont liées avec des éléments de liaison sur la surface de la micropuce.

14. Procédé selon la revendication 13, **caractérisé en ce que** les éléments de liaison sont des éléments de liaison fonctionnalisés par des groupes thiols.

15. Procédé selon l'une des revendications précédentes 3 à 14, **caractérisé en ce que** les éléments des tags utilisés pour le marquage des biomolécules et/ou des molécules identifiant les modifications sont des métaux.

16. Procédé selon la revendication 15, **caractérisé en ce que** le métal utilisé pour le marquage des biomolécules et/ou des molécules identifiant les modifications est un lanthanide.

17. Procédé selon l'une des revendications précédentes 2 à 16, **caractérisé en ce que** le tag est un complexe chélate.

18. Procédé selon la revendication 17, **caractérisé en ce que** le complexe chélate se compose d'un métal de lanthanide et d'un ligand de type DTPA, dérivé de DTPA, DOTA ou dérivé de DOTA.

19. Procédé selon l'une des revendications précédentes, **caractérisé en ce que** l'espèce identifiant les biomolécules est également une biomolécule.

20. Procédé selon l'une des revendications précédentes, **caractérisé en ce que** la molécule identifiant les modifications est également une biomolécule.

21. Kit de diagnostic pour l'identification et la quantification des protéines selon un procédé des revendications 1 à 20, comprenant :
(a) une micropuce dotée d'espèces identifiant les protéines ou une micropuce et des espèces identifiant les protéines à fixer sur la micropuce,
(b) des réactifs de marquage pour le marquage des protéines,
(c) des espèces qui identifient les modifications post-traductionnelles et des réactifs de marquage pour le marquage des espèces qui identifient les modifications post-traductionnelles ou des espèces déjà marquées qui identifient les modifications post-traductionnelles, **caractérisé en ce que**
les réactifs de marquage sont à chaque fois des tags.

22. Kit de diagnostic selon la revendication 21, **caractérisé en ce qu'**il comprend un élément de liaison pour la fixation des espèces identifiant les protéines.

23. Utilisation du kit de diagnostic selon la revendication 21 ou 22 pour l'analyse des protéines.
